# EUROPÄISCHE PATENTSCHRIFT

(11) **EP 1 278 484 B1**
(45) Veröffentlichungstag und Bekanntmachung des Hinweises auf die Patenterteilung: **12.01.2005**
(21) Anmeldenummer: 00943916.7
(22) Anmeldetag: 28.06.2000
(51) Int. Cl.: A61F 2/34

(54) **SANDWICH-INSERT AUS KERAMIK FÜR EIN KÜNSTLICHES HÜFTGELENK**
CERAMIC SANDWICH INSERT FOR AN ARTIFICIAL HIP JOINT
INSERTION EN SANDWICH EN CERAMIQUE POUR ROTULE ARTIFICIELLE

(30) Priorität: 15.07.1999 DE 19933206; 08.01.2000 DE 10000521
(43) Veröffentlichungstag der Anmeldung: 29.01.2003
(73) Patentinhaber: CeramTec AG Innovative Ceramic Engineering, 73207 Plochingen (DE)
(72) Erfinder: BUNZ, Uwe, D-72649 Wolfschlugen (DE); HOCH, Ernst, D-73274 Notzingen (DE); PFAFF, Hans-Georg, D-73760 Ostfildern (DE); RACK, Robert, D-68165 Mannheim (DE)
(74) Vertreter: Scherzberg, Andreas, Dr.
(86) Internationale Anmeldenummer: PCT/EP2000/005992
(87) Internationale Veröffentlichungsnummer: WO 2001/005338

(56) Entgegenhaltungen:
- EP-A- 0 144 209
- EP-A- 0 453 694
- EP-A- 0 554 214
- EP-A- 0 630 624
- EP-A- 0 722 703
- DE-A- 19 640 747
- DE-C- 3 535 959
- FR-A- 2 628 967
- FR-A- 2 668 055
- US-A- 3 818 512

## Beschreibung

Die Erfindung betrifft ein Sandwich-Insert mit einer inneren Gleitschale aus Keramik, die von einem Kunststoffmantel umschlossen ist, zum Einsetzen in eine äußere Metallschale eines künstlichen Hüftgelenks.

Ein Sandwich-Insert gemäß dem Oberbegriff von Anspruch 1 ist aus der EP-A-0 453 694 bekannt.

Ein künstliches Hüftgelenk besteht in der Regel aus einer Gleitschale, die direkt oder über einen Kunststoffmantel in eine äußere Metallschale eingesetzt ist. Diese Metallschale wird in den Beckenknochen implantiert. Die Kombination Gleitschale mit Kunststoffmantel wird als Sandwich-Insert bezeichnet.

In den Oberschenkelknochen wird ein Schaft implantiert, auf dem ein Kugelkopf angeordnet ist, der in der Gleitschale artikuliert.

Immer wieder kann es bei künstlichen Hüftgelenken zum Anschlagen des Kugelkopfschaftes an die Hüftgelenkspfanne kommen. Sind die Anschlagkräfte groß genug, kann dies zur Auflösung des mechanischen Hüftgelenkpfannenverbundes führen. Insbesondere Sandwich-Pfannensysteme sind hier gefährdet, da das überwiegend verwendete PE (Polyethylen) nur einen unzureichenden Widerstand gegen diese Anschlagkräfte aufbieten kann.

Sandwich-Inserts werden auf verschiedene Art und Weise hergestellt.

Bei einem System wird die keramische Gleitschale bzw. das Insert mit Kunststoff umspritzt, wobei an der Gleitschale Rücksprünge angeordnet sind. Nachteilig hieran sind die schlechteren Polyethylen (PE) - Eigenschaften, die durch die Erwärmung desselben resultieren. Ferner erfolgt ein Thermoschock für die keramische Gleitschale. Neben dem Umspritzaufwand durch die Spritzform und der Handhabung der heißen Teile ist der große Bauraumbedarf von Nachteil.

Bei einem alternativen System wird die Gleitschale durch eine konische Klemmung im Kunststoffmantel verankert, bei teilweisen geringen Festigkeiten des Verbundbauteils. Nachteilig ist auch hier der große Bauraumbedarf.

Bevorzugt wird auch das warme Einpressen der Gleitschale in den Kunststoffmantel verwendet. Hierbei treten jedoch teilweise zu geringe Festigkeiten des Verbundbauteils auf. Außerdem sind enge Toleranzen wegen der Preßverbindung zu beachten.

Der Erfindung liegt die Aufgabe zugrunde, ein Sandwich-Insert nach dem Oberbegriff des Anspruchs 1 so zu verbessern, daß eine hohe Umschlagfestigkeit bei kleinem Bauraumbedarf erreicht ist.

Erfindungsgemäß wird diese Aufgabe durch das Sandwich-Insert gemäß Anspruch 1 gelöst. Der Zapfen macht nahezu keine Bauraumvergrößerung erforderlich.

Vorteilhafterweise ist der Zapfen auf der Mittelachse bzw. der Rotationsachse der Gleitschale angeordnet. Die axiale Länge des Zapfens von 1 - 8 mm, bevorzugt von ca. 2 mm ist zur Erhöhung der Umschlagfestigkeit ausreichend.

In bevorzugter Ausführungsform ist der Zapfen mittels einer Passung im Kunststoffmantel angeordnet. Dies kann eine Übermaßpassung, Übergangspassung oder eine enge Spielpassung sein, je nach der gewünschten Widerstandskonfiguration.

Der Zapfen kann den Kunststoffmantel vollständig durchragen, in ihn hineinragen oder aber mindestens teilweise vom Kunstoffmantel umschlossen sein. Bevorzugt ist er auch vollständig vom Kunststoffmantel umschlossen.

Der Querschnitt des Zapfens bildet ein N - Flach mit N = 2,4,5 oder 6. Alternativ kann der Querschnitt des Zapfens auch ein Polygon bilden oder oval sein.

Die Gleitschale weist bevorzugt auf ihrer Außenseite eine sphärische oder eine abgestufte Bauform auf. Sphärische Bauformen haben einen sehr kleinen Bauraumbedarf.

In vorteilhafter Ausführungsform umklammert der Kunststoffmantel die Gleitschale an ihrem offenen Ende.

Hergestellt wird das Sandwich-Insert bevorzugt durch Einpressen der Gleitschale in den Kunststoffmantel.

Weitere Merkmale der Erfindung ergeben sich aus den Figuren, die nachfolgend beschrieben sind. Es zeigt:
- Fig. 1: ein erfindungsgemäßes Sandwich-Insert in sphärischer Bauform,
- Fig. 2: ein erfindungsgemäßes Sandwich-Insert in abgestufter Bauform und
- Fig. 3: vorteilhafte Ausgestaltungen der Querschnitte des Zapfens.

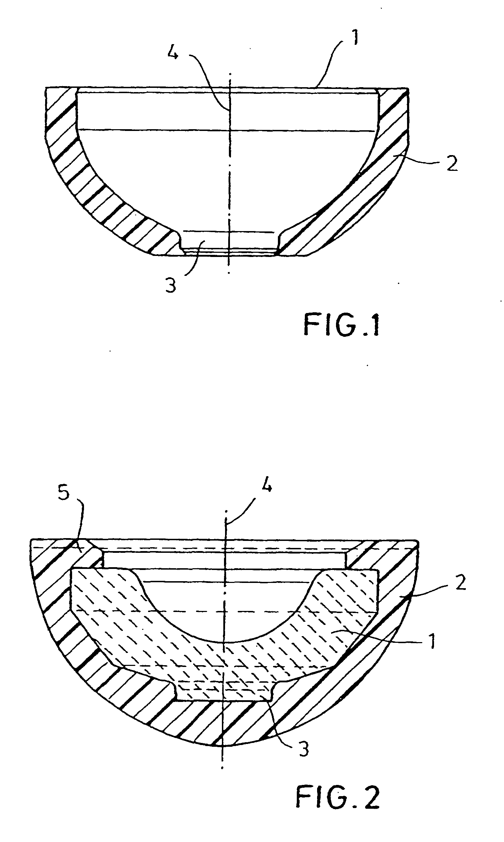

Fig. 1 zeigt ein Sandwich-Insert mit einer Gleitschale 1 in sphärischer Bauform. Bei der Herstellung wird diese Gleitschale 1 in den Kunststoffmantel 2 gepreßt. Der Kunststoffmantel 2 besteht bevorzugt aus Polyethylen (PE). Der obere Rand der Gleitschale ist bündig mit dem oberen Rand des Kunststoffmantels 2 ausgeführt. Zur Erhöhung der Umschlagfestigkeit, d.h. auch zur besseren Verankerung der Gleitschale 1 im Kunststoffmantel 2 ist an dem der Öffnung abgewandten Ende der Gleitschale auf der Rotationsachse bzw. Mittelachse 4 ein Zapfen 3 angeordnet. Dieser Zapfen 3 ragt in den Kunststoffmantel 2 in dieser Ausführungsform hinein.

Fig. 2 zeigt eine alternative Ausführungsform mit einer abgestuften Bauform der Gleitschale 1 auf ihrer Außenseite. Hier ist ebenfalls ein Zapfen 3 auf der Mit-telachse 4 angeordnet, der hier vollständig vom Kunststoffmantel 2 umschlossen ist. Die axiale Länge des Zapfens 3 liegt bei ca. 2 mm.

Da bei der Herstellung die Gleitschale 1 in den Kunststoffmantel 2 eingepreßt wird, ist der Zapfen 3 mittels einer Passung im Kunststoffmantel 2 eingesetzt.

An ihrem offenen Ende umklammert der Kunststoffmantel 2 die Gleitschale 1, wodurch die Befestigung verbessert ist. Der auf der Oberseite der Gleitschale 1 aufliegende Kragen 5 des Kunststoffmantels 2 bedeckt nahezu die Hälfte des Oberrandes.

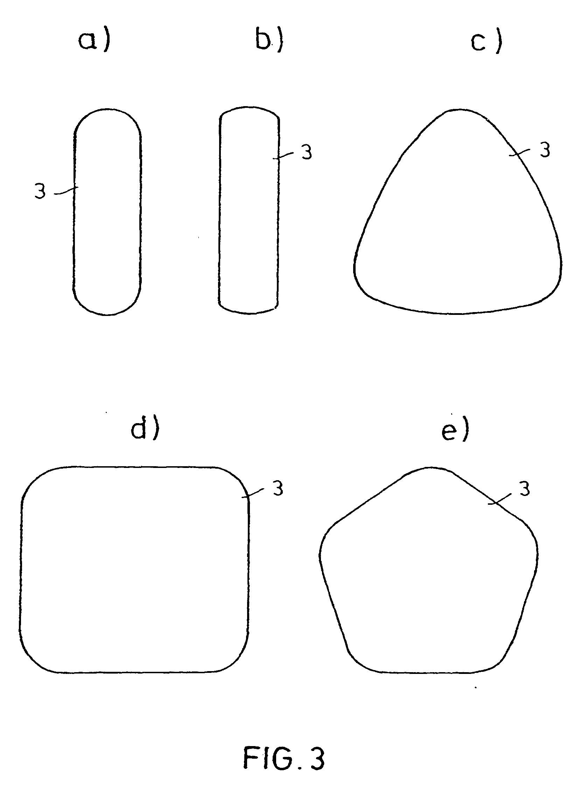

Fig. 3 zeigt an Querschnitten die verschiedenen Ausgestaltungen des Zapfens 3. Es ist jeweils ein Querschnitt des Zapfens 3 senkrecht zur Mittelachse 4 gezeigt.

Fig. 3a zeigt einen ovalen Querschnitt um die Rotationsfestigkeit zu erhöhen, Fig. 3b ein Zweiflach und Fig. 3c einen polygonalen Querschnitt. Dieser hat den Vorteil der drehenden Herstellbarkeit bei relativ hoher Kunststoffverdrängung.

Fig. 3d zeigt ein 4-Flach, bzw. einen quadratischen Zapfen 3 und Fig. 3e ein 5-Flach als Zapfen 3. Vorteilhaft ist auch noch ein 6-Flach als Zapfenquerschnitt.

## Patentansprüche

1. Sandwich-Insert mit einer inneren Gleitschale (1) aus Keramik, die von einem Kunststoffmantel (2) umschlossen ist, zum Einsetzen in eine äußere Metallschale eines künstlichen Hüftgelenks, wobei die Gleitschale (1) an ihrem der Öffnung abgewandten äußeren Ende einen Zapfen (3) aufweist, **dadurch gekennzeichnet, daß** der Querschnitt des Zapfens (3) ein N-Flach bildet mit bevorzugt N=2,4,5 oder 6 oder der Querschnitt des Zapfens (3) ein Polygon bildet oder der Querschnitt des Zapfens (3) oval ist und der Zapfen (3) eine axiale Länge von 1 - 8 mm, bevorzugt von ca. 2 mm, hat.

2. Sandwich-Insert nach Anspruch 1, **dadurch gekennzeichnet, daß** der Zapfen (3) auf der Mittelachse (4) der Gleitschale (1) angeordnet ist.

3. Sandwich-Insert nach Anspruch 1 oder 2, **dadurch gekennzeichnet, daß** der Zapfen (3) mittels einer Passung im Kunststoffmantel (2) angeordnet ist.

4. Sandwich-Insert nach einem der Ansprüche 1 bis 3, **dadurch gekennzeichnet, daß** der Zapfen (3) in den Kunststoffmantel (2) hineinragt oder ihn vollständig durchragt.

5. Sandwich-Insert nach einem der Ansprüche 1 bis 3, **dadurch gekennzeichnet, daß** der Zapfen (3) mindestens teilweise vom Kunststoffmantel (2) umschlossen ist.

6. Sandwich-Insert nach einem der Ansprüche 1 bis 5, **dadurch gekennzeichnet, daß** die Gleitschale (1) auf ihrer Außenseite eine sphärische oder eine abgestufte Bauform aufweist.

7. Sandwich-Insert nach einem der Ansprüche 1 bis 6, **dadurch gekennzeichnet, daß** der Kunststoffmantel (2) die Gleitschale (1) an ihrem offenen Ende umklammert.

8. Sandwich-Insert nach einem der Ansprüche 1 bis 7, **dadurch gekennzeichnet, daß** die Gleitschale (1) in den Kunststoffmantel (2) eingepreßt ist.

## Claims

1. Sandwich insert having an inner ceramic sliding cup (1), which is surrounded by a plastics cover (2), for insertion into an outer metal shell of an artificial hip joint, wherein the sliding cup (1) has a stud (3) at its outer end that is remote from the opening, **characterised in that** the cross section of the stud (3) forms an n-sided body, preferably with n = 2, 4, 5 or 6, or the cross section of the stud (3) forms a polygon or the cross section of the stud (3) is oval and the stud (3) has an axial length of 1 - 8 mm, preferably approximately 2 mm.

2. Sandwich insert according to claim 1, **characterized in that** the stud (3) is arranged on the central axis (4) of the sliding cup (1).

3. Sandwich insert according to claim 1 or 2, **characterized in that** the stud (3) is arranged in the plastics cover (2) by means of a fit.

4. Sandwich insert according to one of claims 1 to 3, **characterized in that** the stud (3) projects into the plastics cover (2) or projects completely through it.

5. Sandwich insert according to one of claims 1 to 3, **characterized in that** the stud (3) is surrounded at least in part by the plastics cover (2).

6. Sandwich insert according to one of claims 1 to 5, **characterized in that** the sliding cup (1) is of a spherical or a stepped structural form on its outside.

7. Sandwich insert according to one of claims 1 to 6, **characterized in that** the plastics cover (2) embraces the sliding cup (1) at its open end.

8. Sandwich insert according to one of claims 1 to 7, **characterized in that** the sliding cup (1) is pressed into the plastics cover (2).

## Revendications

1. Insert de type sandwich avec une cupule (1) intérieure en céramique qui est entourée d'une enveloppe (2) en matière plastique, destiné à être inséré dans une coquille en métal d'une articulation artificielle de hanche, la cupule (1) comportant un tenon (3) à son extrémité extérieure éloignée de l'ouverture, **caractérisé par le fait que** la section transversale du tenon (3) forme un élément à N pans avec N= 2, 4, 5 ou 6, ou la section transversale du tenon (3) forme un polygone ou la section transversale du tenon (3) est ovale et que le tenon (3) a une longueur axiale de 1 - 8 mm, de préférence d'environ 2 mm.

2. Insert de type sandwich selon la revendication 1, **caractérisé par le fait que** le tenon (3) est disposé sur l'axe médian (4) de la cupule (1).

3. Insert de type sandwich selon la revendication 1 ou 2, **caractérisé par le fait que** le tenon (3) est disposé dans l'enveloppe (2) en matière plastique par un ajustement.

4. Insert de type sandwich selon une des revendications 1 à 3, **caractérisé par le fait que** le tenon (3) fait saillie dans l'enveloppe (2) en matière plastique ou traverse celle-ci.

5. Insert de type sandwich selon une des revendications 1 à 3, **caractérisé par le fait que** le tenon (3) est au moins partiellement entouré de l'enveloppe (2) en matière plastique.

6. Insert de type sandwich selon une des revendications 1 à 5, **caractérisé par le fait que** la cupule (1) présente extérieurement une forme sphérique ou une forme étagée.

7. Insert de type sandwich selon une des revendications 1 à 6, **caractérisé par le fait que** l'enveloppe (2) en matière plastique enserre la cupule (1) à son extrémité ouverte.

8. Insert de type sandwich selon une des revendications 1 à 7, **caractérisé par le fait que** la cupule est emmanchée par force dans l'enveloppe (2) en matière plastique.
